# EUROPEAN PATENT APPLICATION

(11) **EP 4 477 083 A1**
(43) Date of publication of application: **18.12.2024**
(21) Application number: 23874561.6
(22) Date of filing: 28.08.2023
(51) Int. Cl.: A01N 59/00, A01N 25/02, A01P 1/00, A01P 3/00, A61L 2/18, A61L 9/01, A61L 9/14, F24F 8/24, A61L 101/22

(54) **DECONTAMINATION SOLUTION AND DEVICE AND METHOD FOR DECONTAMINATION USING SAME**

(30) Priority: 05.10.2022 JP 2022160801
(71) Applicant: Airex Co., Ltd., Nagoya-shi Aichi 453-0015 (JP)
(72) Inventor: OGATA, Yoshitaka, Nagoya-shi Aichi 453-0015 (JP); FUTAMURA Haruka, Nagoya-shi Aichi 453-0015 (JP); GUO, Zhiqiang, Nagoya-shi Aichi 453-0015 (JP); KITANO, Tsukasa, Nagoya-shi Aichi 453-0015 (JP); UKIURA, Ayumi, Nagoya-shi Aichi 453-0015 (JP); ITO, Narumi, Nagoya-shi Aichi 453-0015 (JP); KUREBAYASHI, Kohei, Nagoya-shi Aichi 453-0015 (JP); AOI, Keito, Nagoya-shi Aichi 453-0015 (JP); OKUMURA, Suzune, Nagoya-shi Aichi 453-0015 (JP); OSHIMA, Mitsutoshi, Nagoya-shi Aichi 453-0015 (JP); KAWASAKI, Koji, Nagoya-shi Aichi 453-0015 (JP)
(74) Representative: Kraus & Lederer PartGmbB
(86) International application number: PCT/JP2023/030860
(87) International publication number: WO 2024/075429

(57) **Abstract**

The present invention provides a decontamination solution capable of preventing charging of a mist for decontamination and preventing excessive wetting and liquid dripping on a wall surface inside a working chamber to be decontaminated when the inside of a working chamber such as an isolator is decontaminated using a mist for decontamination composed of hydrogen peroxide, and a device and a method for decontamination using the same.

The decontamination solution is composed of a hydrogen peroxide solution where a substance increasing the electric conductivity of an aqueous solution is dissolved. The substance is preferably an electrolyte and more preferably an inorganic salt or an organic acid. The decontamination solution preferably has an electric conductivity of 14 µS/cm or more. The decontamination solution preferably has a residue on evaporation of 30 ppm or less. The device and the method for decontamination discharge the decontamination solution toward the inside of a room to be decontaminated as a mist for decontamination, thereby decontaminating the inside of the room to be decontaminated.

## Description

### TECHNICAL FIELD

The present invention relates to a decontamination solution used for decontaminating a working chamber such as not only an isolator, a RABS, and a clean room, but also a passing room and a pass box associated therewith by generating a mist for decontamination in the working chamber, and a device and a method for decontamination using the same.

### BACKGROUND ART

In manufacturing settings for pharmaceutical or food products or in the clinical environment such as operating rooms, the indoor working area must inevitably be kept sterile. Particularly in cases where clean rooms as a working chamber for manufacturing pharmaceutical products are decontaminated, advanced decontamination validation needs to be accomplished in accordance with Good Manufacturing Practice (GMP).

In recent years, hydrogen peroxide gas has widely been used to decontaminate a working chamber requiring sterile environment. Advantageously, hydrogen peroxide gas has a strong sterilization effect, and is inexpensively available and effectively utilized as an environmentally-friendly decontamination gas that is ultimately resolved into oxygen and water.

Meanwhile, the following patent document 1 describes that the decontamination effect by hydrogen peroxide is provided by a condensed film of a hydrogen peroxide solution that condenses on the surface of an object to be decontaminated. Inventors of the present invention found that a fine mist of hydrogen peroxide solution (hereinafter referred to as a "mist for decontamination"), instead of a hydrogen peroxide gas, is supplied to a working chamber to be decontaminated to achieve efficient decontamination using the least possible amount of decontamination solution, and suggests the technique as described in the following patent document 2.

### CITATION LIST

### PATENT LITERATURE

Patent Document 1: JP-A-61-004543
Patent Document 2: JP-A-2020-156970

### SUMMARY OF INVENTION

### TECHNICAL PROBLEM

A water particle, once mechanically and microscopically segmentalized, is charged, which is known as "Lenard effect". Generally, conversion of a liquid having a small electric conductivity such as ultrapure water (electric conductivity < 0.1 µS/cm) into a mist with a two-fluid nozzle or an ultrasonic atomizer segmentalizes the orientation dipole of the liquid, and the resulting liquid droplets are charged in a stronger manner as they are smaller. In the present invention, a hydrogen peroxide solution is used as a decontamination solution, which will be refined into a mist for decontamination, using a two-fluid nozzle or an ultrasonic atomizer. It is found that a hydrogen peroxide solution used as a decontamination solution also has a small electric conductivity like an ultrapure water, and its conversion into a fine mist for decontamination with a two-fluid nozzle or an ultrasonic atomizer causes spray charging as in the ultrapure water, resulting in spray-charged mists for decontamination.

Unfortunately, these spray-charged mists for decontamination electrostatically adhere to a wall surface near a mist discharge port to cause the wall surface to get wet by mist condensation. Such partial and excessive wetting during decontamination of the working chamber generates liquid dripping on the wall surface during decontamination. This liquid dripping causes non-uniform mists for decontamination, resulting in lowered and non-uniform decontamination effects. This also makes aeration insufficient. However, no technology has traditionally been developed for preventing adhesion of mists for decontamination composed of hydrogen peroxide used for decontamination by spray charging.

Thus, the present invention was made in view of the situation to solve the problems, and has an object to provide a decontamination solution capable of preventing charging of a mist for decontamination and preventing excessive wetting and liquid dripping on a wall surface inside a working chamber to be decontaminated when the inside of a working chamber such as an isolator is decontaminated using a mist for decontamination composed of hydrogen peroxide, and a device and a method for decontamination using the same.

### SOLUTION TO PROBLEM

To solve the aforementioned problem, inventors of the present invention have focused on the electric conductivity of a hydrogen peroxide solution used as a decontamination solution to find that a substance capable of adjusting the electric conductivity at a constant value or more is dissolved in a hydrogen peroxide solution to achieve the above object. Based on that technique, the present invention was accomplished.

Specifically, a decontamination solution according to the present invention is, according to description in claim 1,
a decontamination solution used for a device for decontamination, the decontamination solution discharged toward the inside of a room to be decontaminated as a mist for decontamination to decontaminate the inside of the room to be decontaminated, characterized in that
the decontamination solution is composed of a hydrogen peroxide solution where an electrolyte increasing the electric conductivity of an aqueous solution is dissolved, characterized in that
the electrolyte is an organic acid and the electric conductivity of the hydrogen peroxide solution is adjusted at 14 µS/cm or more.

Moreover, the present invention is, according to description in claim 2, the decontamination solution according to claim 1, characterized in that
the electrolyte is an organic acid other than an organic phosphonic acid.

Furthermore, the present invention is, according to description in claim 3, the decontamination solution according to claim 1 or 2, characterized in that
the organic acid is a liquid at normal temperature and has a boiling point of 150 °C or less.

Moreover, the present invention is, according to description in claim 4, the decontamination solution according to claim 3, characterized in that
the decontamination solution has a residue on evaporation of 30 ppm or less.

Moreover, a device for decontamination according to the present invention is, according to description in claim 5,
a device for decontamination by discharging a decontamination solution toward the inside of a room to be decontaminated as a mist for decontamination to decontaminate the inside of the room to be decontaminated, including
a decontamination solution supplying device, a mist generator and a mist discharge port, characterized in that
the decontamination solution supplying device reserves the decontamination solution according to description in claim 4 and supplies the decontamination solution to the mist generator,
the mist generator converts the decontamination solution into a mist to generate a mist for decontamination, and
the mist discharge port is opened to an internal wall surface of the room to be decontaminated to discharge the mist for decontamination toward the inside of the room to be decontaminated.

Moreover, a method for decontamination according to the present invention is, according to description in claim 6,
a method for decontaminating the inside of a room to be decontaminated, including a decontamination step and an aeration step, characterized in that
in the decontamination step, using the device for decontamination according to description in claim 5, the mist generator is allowed to generate the mist for decontamination from the decontamination solution and the mist for decontamination is discharged from the mist discharge port to decontaminate the inside of the room to be decontaminated, and
in the aeration step after the decontamination step, clean air is introduced into the inside of the room to be decontaminated to discharge a mist and a condensed film of the decontamination solution residual inside the room to be decontaminated toward the outside of the room to be decontaminated.

### ADVANTAGEOUS EFFECTS OF INVENTION

According to the above configuration, a decontamination solution according to the present invention is composed of a hydrogen peroxide solution where an electrolyte increasing the electric conductivity of an aqueous solution is dissolved. Also, the electrolyte is preferably an organic acid. Preferably, the organic acid is a liquid at normal temperature and has a boiling point of 150 °C or less. Also, the residue on evaporation of the decontamination solution is preferably 30 ppm or less. Moreover, the electric conductivity of the decontamination solution is preferably adjusted at 14 µS/cm or more.

Accordingly, the present invention can provide a decontamination solution capable of preventing charging of a mist for decontamination and preventing excessive wetting and liquid dripping on a wall surface inside a working chamber to be decontaminated when the inside of a working chamber such as an isolator is decontaminated using a mist for decontamination composed of hydrogen peroxide.

According to the above configuration, a device for decontamination according to the present invention includes a decontamination solution supplying device, a mist generator and a mist discharge port. The decontamination solution supplying device reserves the decontamination solution according to the above configuration and supplies the decontamination solution to the mist generator. The mist generator converts the decontamination solution into a mist to generate a mist for decontamination. The mist discharge port is opened on an internal wall surface of a room to be decontaminated to discharge the mist for decontamination toward the inside of the room to be decontaminated.

Accordingly, the present invention can provide a device for decontamination capable of preventing charging of a mist for decontamination and preventing excessive wetting and liquid dripping on a wall surface inside a working chamber to be decontaminated when the inside of a working chamber such as an isolator is decontaminated using a mist for decontamination composed of hydrogen peroxide.

According to the above configuration, a method for decontamination according to the present invention includes a decontamination step and an aeration step. In the decontamination step, using the device for decontamination according to the above configuration, the mist generator is allowed to generate the mist for decontamination from the decontamination solution and the mist for decontamination is discharged from the mist discharge port to decontaminate the inside of the room to be decontaminated. Next, in the aeration step after the decontamination step, clean air is introduced into the inside of the room to be decontaminated to discharge a mist and a condensed film of the decontamination solution residual inside the room to be decontaminated toward the outside of the room to be decontaminated.

Accordingly, the present invention can provide a method for decontamination capable of preventing charging of a mist for decontamination and preventing excessive wetting and liquid dripping on a wall surface inside a working chamber to be decontaminated when the inside of a working chamber such as an isolator is decontaminated using a mist for decontamination composed of hydrogen peroxide.

### BRIEF DESCRIPTION OF DRAWINGS

FIG. 1 is a conceptual diagram showing that a mist for decontamination discharged from a mist discharge port of a device for decontamination is spray-charged and electrostatically adheres to a wall surface of a room to be decontaminated;
FIG. 2 is a schematic side view showing the inside of an isolator mounted with a device for decontamination according to this embodiment;
FIG. 3 is an appearance front view showing the device for decontamination mounted in FIG. 2;
FIG. 4 (A) is a front view showing the configuration of an ultrasonic atomizer constituting the device for decontamination in FIG. 3 and FIG. 4(B) is a right-side cross-sectional view showing the same;
FIG. 5 is a photo showing the state of a water sensitive test paper disposed around a mist discharge port of the device for decontamination in FIG. 3;
FIG. 6 is a diagram showing a table and a photo of evaluation results indicative of the wetting of a water sensitive test paper according to a spray test of each decontamination solution prepared in Example 1; and
FIG. 7 is an internal perspective view showing the state of an enzyme indicator (EI) disposed inside the isolator.

### DESCRIPTION OF EMBODIMENTS

In the present invention, "mist" is broadly interpreted as the state of a liquid droplet of a decontamination solution refined and floating in the air, the state of a gas and a liquid droplet of a decontamination solution in mixture, the state of a decontamination solution to repeat the change in phase between condensation and evaporation of a gas and a liquid droplet, and the like. In terms of particle size as well, the mist is also broadly interpreted to include mists, fogs, aerosols and liquid droplets, which can be subclassified.

Accordingly, the mist according to the present invention is categorized into a "mist" (the size may be defined as 10 µm or less) or a "fog" (the size may be defined as 5 µm or less), and a mist having a larger particle size.

The decontamination solution, the device and the method for decontamination using the same according to the present invention will be described with reference to embodiments. The present invention is not restricted to the following embodiments.

<<Decontamination solution>>

In the present invention, a hydrogen peroxide solution is used as a decontamination solution, converted into a mist to generate a mist for decontamination. The mist for decontamination is discharged toward the inside of a room to be decontaminated to decontaminate indoors. The concentration of the hydrogen peroxide solution used as a decontamination solution is not particularly restricted, but in general, it is preferably 30 to 35 W/V% in view of hazardous materials in use.

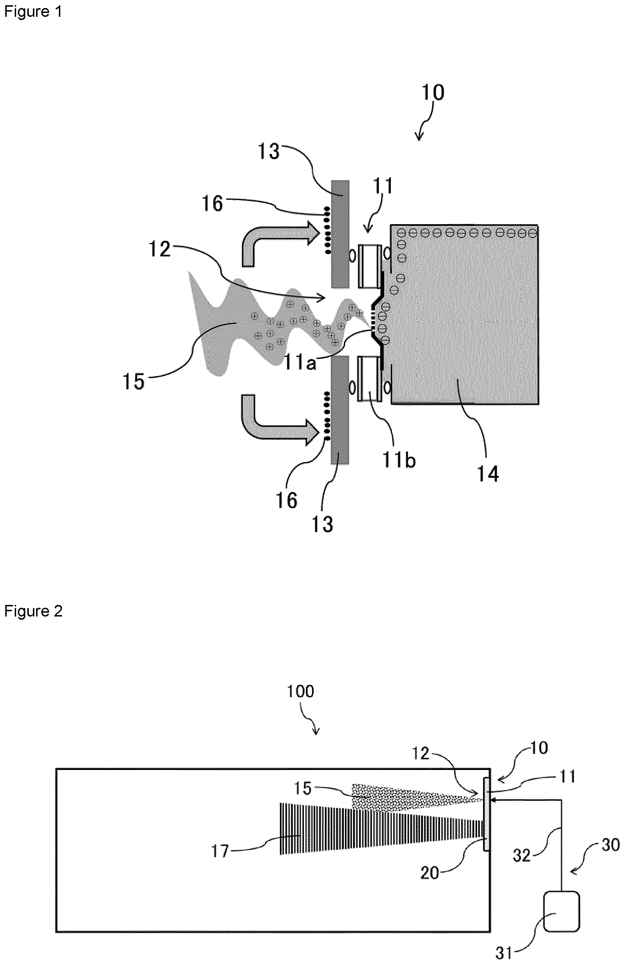

As described above, conversion of a hydrogen peroxide solution into a mist generates spray charging, which allows a mist for decontamination to be strongly charged. The resulting charged mist for decontamination electrostatically adheres inside the room to be decontaminated, particularly to a wall surface near a mist discharge port, causing wetting. FIG. 1 is a conceptual diagram showing that a mist for decontamination discharged from a mist discharge port of a device for decontamination is spray-charged and electrostatically adheres to a wall surface of a room to be decontaminated. In FIG. 1, a device for decontamination 10 includes a decontamination solution supplying device (not shown), a mist generator 11 and a mist discharge port 12, provided on a side wall surface 13 of the room to be decontaminated.

The mist generator 11 is an ultrasonic atomizer to reserve a decontamination solution supplied from the decontamination solution supplying device in a decontamination solution reservoir 14 and convert the same into a mist, i.e. a mist for decontamination 15. The mist generator 11 is composed of a perforated vibration plate 11a for atomizing a decontamination solution and a piezoelectric vibrator 11b for film-vibrating the perforated vibration plate 11a (details later described).

Also, the perforated vibration plate 11a has a front surface directed toward the inside of the room to be decontaminated (leftward of the side wall surface 13 in the figure) and a rear surface disposed toward the inside of the decontamination solution reservoir 14. In addition, a plurality of micropores of the perforated vibration plate 11a passes through the inside of the room to be decontaminated and the decontamination solution reservoir 14. In FIG. 1, the front surface of the perforated vibration plate 11a also serves as the mist discharge port 12.

In FIG. 1, the mist for decontamination 15 discharged from the mist discharge port 12 (perforated vibration plate 11a) is strongly charged by spray charging. Accordingly, the mist for decontamination 15 electrostatically adheres, thereby causing a wetting 16 of the decontamination solution on the front surface of the side wall surface 13. Increases in the wetting 16 generate liquid dripping on the wall surface when the room to be decontaminated is decontaminated. The resulting non-uniform decontamination mists lead to lowered and non-uniform decontamination effects. This also makes aeration insufficient.

The decontamination solution of the present invention has an object to prevent such spray charging from occurring. Inventors of the present invention confirmed that the electric conductivity of a commercially available 35 W/V% hydrogen peroxide solution is about 8 µS/cm on average (details later described). Inventors of the present invention believed that such a low electric conductivity can cause spray charging when a hydrogen peroxide solution is converted into a mist.

Thus, in the present invention, a decontamination solution is prepared by dissolving a predetermined amount of substance increasing the electric conductivity in a hydrogen peroxide solution to adjust the electric conductivity at a predetermined value or more in order to prevent spray charging of a mist for decontamination. The type and amount of a substance increasing the electric conductivity to be added to a hydrogen peroxide solution are not particularly restricted so long as they do not essentially prevent decontamination effects of the hydrogen peroxide solution and successfully secure the quality stability as a decontamination solution.

Illustrative example of a substance increasing the electric conductivity of an aqueous solution includes electrolytes dissociating in the aqueous solution. These electrolytes may include salt, acid, and base. Among the electrolytes in the present invention, inorganic salt or organic acid is particularly effective. Representative inorganic salts used may include chloride, sulfate, nitrate, and carbonate. Representative organic acids used may include formicacid, acetic acid, propionic acid, citric acid, malic acid, lactic acid, succinic acid, tartaric acid, butyric acid, fumaric acid, and peracid such as peracetic acid. These inorganic salts or organic acids may be used alone or in combination with a number of types thereof. Meanwhile, carbon dioxide or ammonia gas may directly be insufflated into an aqueous solution, or carbonated water or ammonia water dissolved in water may be mixed with a hydrogen peroxide solution. However, since these substances can easily evaporate in the form of a gas at normal temperature, the stability of a decontamination solution can be problematic.

Preferably, the organic acid used is a liquid at normal temperature and has a boiling point of 150 °C or less. First, the organic acid that is liquid at normal temperature does not precipitate or solidify inside the room to be decontaminated during decontamination and aeration, leaving no residue inside the room to be decontaminated. A hydrogen peroxide, having a boiling point of approx. 152 °C or less, can readily be discharged from the inside of the room to be decontaminated during aeration. Such an organic acid may preferably be formicacid, acegtic acid, propionic acid, or peracetic acid.

In the present invention, a decontamination solution used is prepared by dissolving a substance increasing the above electric conductivity in a hydrogen peroxide solution. The electric conductivity of the decontamination solution is preferably adjusted at 14 µS/cm or more. With the electric conductivity of the decontamination solution of 14 µS/cm or more, no spray charging of a mist for decontamination occurs, and the mist does not electrostatically adhere on the wall surface of the room to be decontaminated. Alternatively, spray charging is very slight, if any, and a very small amount of a mist for decontamination electrostatically adheres on the wall surface of the room to be decontaminated.

Also, as for the decontamination solution of the present invention, a large amount of residues of a substance increasing the electric conductivity is undesirably found inside the room to be decontaminated that has completed decontamination via a decontamination step and an aeration step. Thus, inventors of the present invention conceived the following solutions to cope with residues on evaporation of a decontamination solution (residue components after evaporation of a decontamination solution at high temperature).

First, a substance increasing the electric conductivity used is most preferably a substance leaving no residue on evaporation. In this regard, such a substance is preferably an organic acid that is a liquid at normal temperature and has a boiling point of 150 °C or less. Meanwhile, even if a slight amount of residue on evaporation is left, a substance is preferably used so long as it expresses required increases in the electric conductivity and has a residue on evaporation of decontamination solution of 30 ppm or less. Further, a substance with a residue on evaporation of 20 ppm or less is more preferably used. The use of a substance having a residue on evaporation of a decontamination solution of 30 ppm or less allows a room to be decontaminated to be used in sterile environment.

### EXAMPLE 1

The correlation between the electric conductivity of a decontamination solution and electrostatic adhesion will be described with reference to examples. In Example 1, citric acid is used as an organic acid for a 35 W/V% hydrogen peroxide solution, and with a variety of amounts to be added, the electric conductivity and the state of electrostatic adhesion are evaluated.

First, the electric conductivity of a commercially available hydrogen peroxide solution was measured. Table 1 shows the results of the electric conductivity of 35 W/V% hydrogen peroxide solutions commercially available from 4 manufacturers, using a conductance meter (Product from HANNA Instruments, Inc., HI98311N). The average electric conductivity of the commercially available 35 W/V% hydrogen peroxide solutions was approx. 8 µS/cm.

**[Table 1]**

| Hydrogen peroxide solution (35 W/V%) | Electric conductivity (µs/cm) |
|---|---|
| Product from Manufacturer A | 7 |
| Product from Manufacturer B | 5 |
| Product from Manufacturer C | 7 |
| Product from Manufacturer D | 12 |
| Average | 7.75 |

Next, the electric conductivity was measured by adding a citric acid aqueous solution (1W/V%) to a 35 W/V% hydrogen peroxide solution from Manufacturer A, with the citric acid concentration in the hydrogen peroxide solution adjusted in the range of 5 to 20 ppm. Table 2 shows the electrical conductivities measured. As for each decontamination solution shown in Table 2, the correlation between the electric conductivity of a decontamination solution and electrostatic adhesion on a wall surface was evaluated, using a device for decontamination (details later described).

**[Table 2]**

| | Citric acid conoentration (ppm) | Electric conductivity (µS/cm) |
|---|---|---|
| Comparative Example 1 | 0 | 7 |
| Example 1-1 | 5 | 9 |
| Example 1-2 | 8 | 11 |
| Example 1-3 | 10 | 13 |
| Example 1-4 | 15 | 14 |
| Example 1-5 | 20 | 16 |

### <<Device for decontamination>>

Herein, a device for decontamination and a room to be decontaminated evaluating the correlation between the electric conductivity of a decontamination solution and electrostatic adhesion on the wall surface will be described based on an embodiment. The present invention is not restricted to the following embodiment.

An embodiment will be described by illustrating an isolator as a room to be decontaminated. FIG. 2 is a schematic side view showing the inside of an isolator mounted with a device for decontamination according to this embodiment. In FIG. 2, an isolator 100 has a stainless chamber having a volume of 1.0m³, the inside of which constitutes a closed space. Inside the isolator 100, a device for decontamination 10 is arranged on an upper side wall surface (right side wall surface in the figure) . In the device for decontamination 10 according to this embodiment, a mist generator 11 used is an ultrasonic atomizer (nebulizer) (details later described). In FIG. 2, related devices such as air-supply and air exhaust devices are omitted.

The ultrasonic atomizer 11 converts a decontamination solution supplied via a decontamination solution supply pipe 32 from a decontamination solution tank 31 of a decontamination solution supplying device 30 into a mist for decontamination 15 to discharge the same from a mist discharge port 12 toward the inside of the isolator 100. In addition, the device for decontamination 10 of this embodiment includes a mist dispersion/diffusion device 20 dispersing and diffusing the mist for decontamination 15 to the inside of the isolator 100 (details later described). The mist dispersion/diffusion device 20 is disposed such that acoustic radiation pressure 17 by ultrasonic vibration from the lower back in the discharge direction acts on the mist for decontamination 15 discharged from the mist discharge port 12 (details later described).

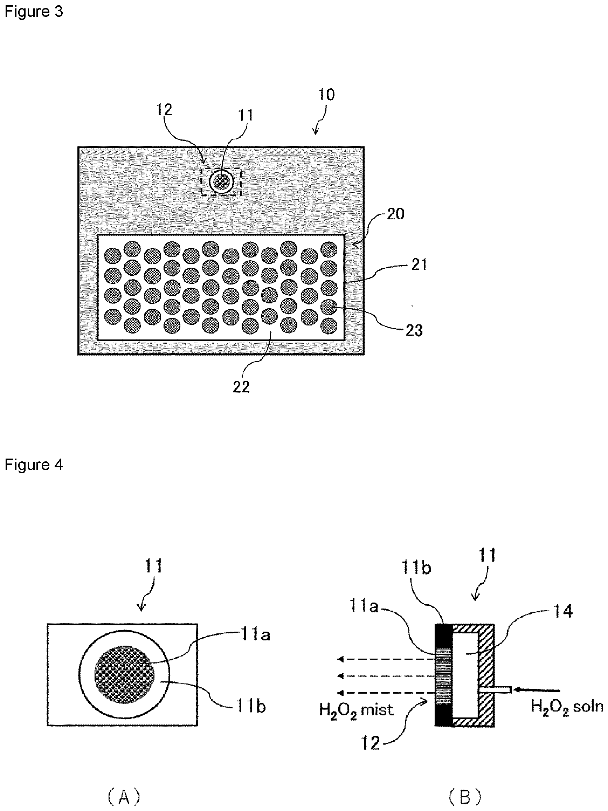

Herein, the ultrasonic atomizer 11 will be described. FIG. 3 is an appearance front view showing the device for decontamination 10. In FIG. 3, the device for decontamination 10 includes the ultrasonic atomizer 11 at an upper portion thereof, with the mist discharge port 12 open to the inside of the isolator 100. There is the mist dispersion/diffusion device 20 below the ultrasonic atomizer 11.

FIG. 4 (A) is a front view showing the configuration of the ultrasonic atomizer constituting the device for decontamination and FIG, 4(B) is a right-side cross-sectional view showing the same. In FIG. 4, the ultrasonic atomizer 11 is composed of a decontamination solution reservoir 14 holding a decontamination solution (H₂O₂ soln) supplied from a decontamination solution supplying device (not shown), a substantially annular disk-shaped perforated vibration plate 11a provided with a plurality of micropores atomizing the decontamination solution held in the decontamination solution reservoir 14, the micropores passing through the perforated vibration plate between the front surface and the rear surface thereof, a piezoelectric vibrator 11b formed of a substantially annular disk in which the perforated vibration plate 11a is subjected to film vibration, and a controller (not shown) controlling the vibration of the piezoelectric vibrator 11b. The perforated vibration plate 11a is affixed to the piezoelectric vibrator 11b so as to cover an internal hole portion of the piezoelectric vibrator 11b.

Also, the perforated vibration plate 11a is attached such that the front surface faces the inside of the room to be decontaminated and the rear surface faces the inside of the decontamination solution reservoir 14, and a plurality of micropores of the perforated vibration plate 11a passes through the inside of the room to be decontaminated and the inside of the decontamination solution reservoir 14. In this embodiment, the front surface of the perforated vibration plate 11a also serves as the mist discharge port 12. In FIG. 4, the perforated vibration plate 11a is disposed so as to discharge the mist for decontamination 15 horizontally from the front surface of the perforated vibration plate 11a, but the configuration is not restricted thereto, and the mist for decontamination may be discharged downward or upward, depending on the position of the plate disposed.

Subsequently, the mist dispersion/diffusion device 20 and the operation thereof will be described. In FIG. 3, the mist dispersion/diffusion device 20 includes an ultrasonic vibrating board 21. The ultrasonic vibrating board 21 is disposed such that acoustic radiation pressure 17 by ultrasonic vibration from the rear of a lower portion in the discharge direction acts on the mist for decontamination 15 discharged from the ultrasonic atomizer 11.

Herein, the structure and operation of the ultrasonic vibrating board 21 will be described. In FIG. 3, the ultrasonic vibrating board 21 includes a base and a plurality of transmitters. In this embodiment, a base 22 is used, and the transmitter used is an ultrasonic transmitter 23. In this embodiment, a plurality of ultrasonic transmitters 23 is disposed on the base 22 so as to be uniform in transmission direction of a vibrating surface thereof (toward the viewer in the figure). The number of the ultrasonic transmitters 23 is not particularly restricted.

In this embodiment, an ultra directional ultrasonic transmitter 23 is used. Specifically, an ultrasonic transmitter (DC12V, 50mA) of frequency modulation system for transmitting an ultrasound whose frequency is around 40 KHz is used. The type, size, structure and output of the ultrasonic transmitter 23 are not particularly restricted. In the present invention, the ultrasonic vibrating board 21 is not restricted to an ultrasonic transmitter, and the ultrasonic generation mechanism, frequency range and output are not particularly restricted.

In this embodiment, a plurality of ultrasonic transmitters 23 is arranged so as to be uniform in transmission direction of the vibrating surface, and the transmitters are operated in the same phase to mutually amplify ultrasounds from the plurality of ultrasonic transmitters 23 in the front direction and mutually cancel out ultrasounds from the plurality of ultrasonic transmitters 23 in the lateral direction. Consequently, the ultrasonic transmitters 23 arranged on the base 22 are subjected to ultrasonic vibration to generate a significantly directional sound flow traveling in the air from each of the vibrating surfaces in the vertical direction. A controller (not shown) controls the frequency, output, and transmission time of an ultrasonic transmitter 23, and the pressure on the mist for decontamination 15 by the acoustic radiation pressure 17 can be changed in intermittent operation or stronger/weaker operation of ultrasonic transmission.

### <<Method for decontamination>>

Next, a method for decontamination according to the present invention will be described, and the correlation between the electric conductivity of each of the decontamination solutions prepared in the above Example 1 by the method for decontamination and the electrostatic adhesion on a wall surface was evaluated.

The method for decontamination of the present invention includes a decontamination step and an aeration step, which is similar to a conventional method for decontamination. In this embodiment, a decontamination solution was inputted to an isolator 100 (volume: 1.0 m³) including the above device for decontamination 10, with an input speed of 1.0 g/min (equivalent to 10 g/m³) for 10 minutes in the decontamination step. In the isolator during the decontamination step, the temperature was 23 °C, and the humidity was 50 %. In the following aeration step, mists and condensed films of the decontamination solution residual in the isolator were removed by a 25-minute aeration.

Using each of the decontamination solutions of Examples 1-1 to 1-5 prepared in the above Example 1 and a decontamination solution of Comparative Example 1, the correlation between the electric conductivity and the electrostatic adhesion on a wall surface was evaluated.

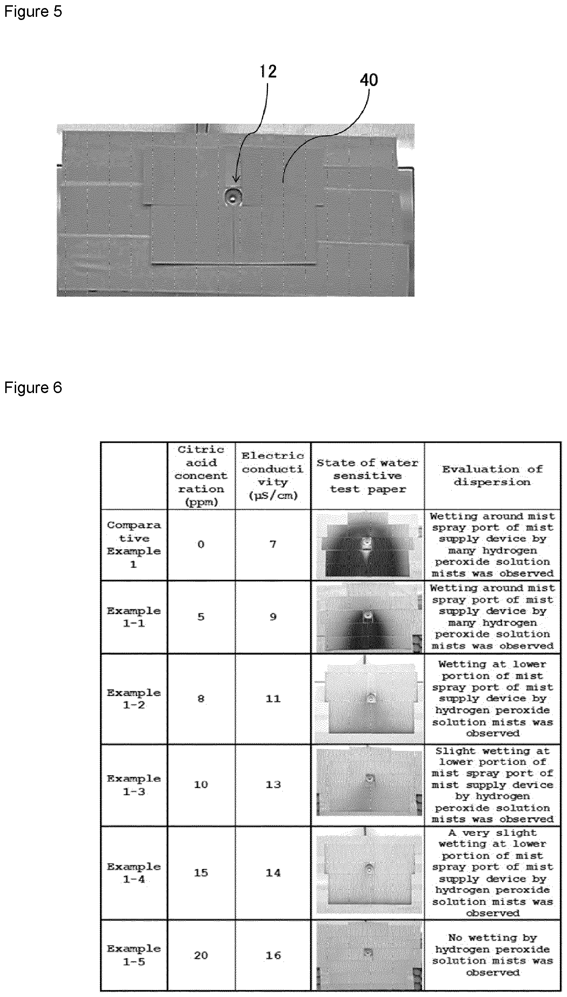

FIG. 5 is a photo showing the state of a water sensitive test paper disposed around a mist discharge port of the above device for decontamination. In FIG. 5, the mist discharge port 12 is surrounded by a plurality of water sensitive test papers 40. The water sensitive test paper (Product from Spraying Systems Co., Japan) is yellow in dried state, but turns blue in water, and is bluer as it is wetter. This testing allows for evaluation of the adhesion of the mist for decontamination on the test paper and the state of adhesion.

FIG. 6 is a diagram showing a table and a photo of evaluation results indicative of the wetting of a water sensitive test paper according to a spray test of each decontamination solution prepared in Example 1. In FIG. 6, in Comparative Example 1 (7 µS/cm), the wetting around the mist discharge port by many mists for decontamination was observed. Also, in Example 1-1 (9 µS/cm) , the wetting around the mist discharge port (at a lower portion, in particular) by many mists for decontamination was observed. In Example 1-2 (11 µS/cm), the wetting at a lower portion of the mist discharge port by mists for decontamination was observed. In Example 1-3 (13 µS/cm), a slight wetting at the lower portion of the mist discharge port by mists for decontamination was observed.

Compared to these Examples, in Example 1-4 (14 µS/cm), a very slight wetting at the lower portion of the mist discharge port by mists for decontamination was observed. In Example 1-5 (16 µS/cm), no wetting around the mist discharge port by mists for decontamination was observed. These evaluations and results find that by adjusting the electric conductivity of a 35 W/V% hydrogen peroxide solution at 14 µS/cm or more, no or little wetting by dispersion of mists for decontamination.

### EXAMPLE 2

In this Example 2, using 2 types of decontamination solutions (35 W/V% hydrogen peroxide solution) having different electrical conductivities, decontamination effects inside the isolator were evaluated.

In this embodiment 2, decontamination effects were evaluated by performing a decontamination step and an aeration step in the isolator 100 (volume: 1.0 m³) including the above device for decontamination 10. In Example 2 (16 µS/cm), the decontamination solution prepared in the above Example 1 (Example 1-5) is used as 2 types of decontamination solutions having different electrical conductivities. In addition, the decontamination solution (Comparative Example 1) prepared in the above Example 1 was used in Comparative Example 2 (7 µS/cm).

Decontamination effects were evaluated by an enzyme indicator (EI) . EI is used for evaluating a decontamination intensity by measuring the residual enzymatic activity after a test and the emission intensity by a luciferase assay. The current EI removes culture operations in conventional biological indicator (BI) and reduces the duration of operations to measure the decontamination intensity. EI's comparative equality with BI was recently confirmed and the EI technique has proactively been used.

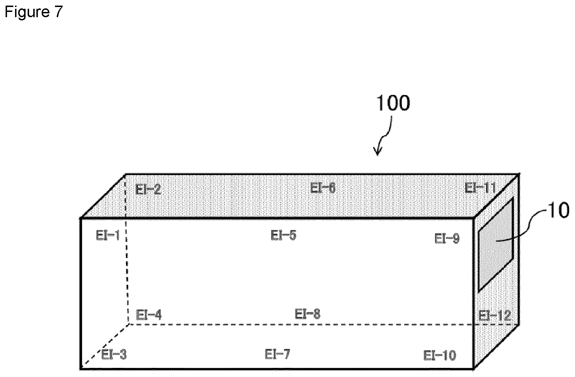

Specifically, the log spore reduction (LRD) value was calculated by the logarithmic decrement of fungi from the EI's fluorescence intensity after decontamination, and the decontamination intensity distribution inside the isolator was evaluated. FIG. 7 is an internal perspective view showing the state of an enzyme indicator (EI) disposed inside an isolator. As shown in FIG. 7, 12 EIs (EI-1 to EI-12) were disposed inside the isolator 100.

In the decontamination step, a decontamination solution was inputted with an input speed of 1.0 g/min for 10 minutes (equivalent to 10 g/m³). In the isolator during the decontamination step, the temperature was 23 °C, and the humidity was 50 %. In the following aeration step, the hydrogen peroxide residual in the isolator was removed by a 25-minute aeration. Thereafter, the EIs were removed to evaluate LRD values from the emission intensity. Table 3 shows the evaluation of the LRD values.

**[Table 3]**

| EI's location | LRD value | |
|---|---|---|
| | Comparative Example 2 | Example 2 |
| | 35 W/V% hydrogen peroxide solution (7 µS/cm) | 35 W/V% hydrogen peroxide solution (16 µS/cm) |
| EI-1 | 4.5 | 6.1 |
| EI-2 | 4.7 | 6.0 |
| EI-3 | 4.8 | 6.4 |
| EI-4 | 4.9 | 6.2 |
| EI-5 | 5.0 | 6.5 |
| EI-6 | 5.1 | 6.4 |
| EI-7 | 5.2 | 6.0 |
| EI-8 | 5.3 | 6.2 |
| EI-9 | 5.3 | 6.9 |
| EI-10 | 5.4 | 7.0 |
| EI-11 | 5.6 | 6.3 |
| EI-12 | 5.7 | 6.6 |
| Average | 5.1±0.36 | 6.4±0.32 |

As shown in Table 3, the LRD values in Example 2 (16 µS/cm) show overall high decontamination intensity distribution and high average decontamination intensity, compared to the LRD values in Comparative Example 2 (7 µS/cm) . The resulting reduction in electrostatic adhesion of mists for decontamination leads to effective use of mists for decontamination for decontamination. Another finding is that there are small variations in decontamination intensity, and decontamination effects at any location in the isolator are favorable.

As described above, the present invention can provide a decontamination solution capable of preventing charging of a mist for decontamination and preventing excessive wetting and liquid dripping on a wall surface inside a working chamber to be decontaminated when the inside of a working chamber such as an isolator is decontaminated using a mist for decontamination composed of hydrogen peroxide, and a device and a method for decontamination using the same.

The present invention is achieved not only by the above embodiment, but also by the following various alternatives.
(1) In each of the above embodiments, an isolator is defined as a room to be decontaminated, but the isolator is not restricted to that, and may be a RABS, a clean room, a passing room, a pass box, or a combination thereof.
(2) In each of the above embodiments, the mist generator used is an ultrasonic atomizer, but it is not restricted to that. The mist generator may be, for example, a single-fluid spray nozzle directly converting a liquid into a mist, a piezo-high pressure injection device, a dipping-type ultrasonic atomizer, a disk-type atomizer, and a disk-mesh-type atomizer. In addition, the mist generator may be an ejector converting a liquid into a mist with high-pressure air or the like or a two-fluid spray nozzle.
(3) In each of the above embodiments, the mist generator used is an ultrasonic atomizer to convert a decontamination solution into a mist for decontamination, but it is not restricted to that. For example, a mist generator may be a primary mist generator and a secondary mist generator in combination to convert a decontamination solution into a finer mist for decontamination.
(4) In each of the above embodiments, a device for decontamination includes a mist dispersion/diffusion device, but the configuration is not restricted to that, and no mist dispersion/diffusion device may be used.

### REFERENCE SIGNS LIST

10...Device for decontamination, 11...Mist generator,
11a...Perforated vibration plate, 11b...Piezoelectric vibrator,
12...Mist discharge port, 13...Side wall surface,
14...Decontamination solution reservoir, 15...Mist for decontamination,
16...Wetting of decontamination solution, 17...Acoustic radiation pressure, 20...Mist dispersion/diffusion device,
21...Ultrasonic vibrating board, 22...Base, 23...Ultrasonic transmitter,
30...Decontamination solution supplying device,
31...Decontamination solution tank, 32... Decontamination solution supplying pipe,
40...Water sensitive test paper, 100...Isolator.

## Claims

1. A decontamination solution used for a device for decontamination, the decontamination solution discharged toward the inside of a room to be decontaminated as a mist for decontamination to decontaminate the inside of the room to be decontaminated, wherein
said decontamination solution is composed of a hydrogen peroxide solution where an electrolyte increasing the electric conductivity of an aqueous solution is dissolved, wherein
the electrolyte is an organic acid and the electric conductivity of said hydrogen peroxide solution is adjusted at 14 µS/cm or more.

2. The decontamination solution according to claim 1, wherein
said electrolyte is an organic acid other than an organic phosphonic acid.

3. The decontamination solution according to claim 1 or 2, wherein
said organic acid is a liquid at normal temperature and has a boiling point of 150 °C or less.

4. The decontamination solution according to claim 3, wherein
said decontamination solution has a residue on evaporation of 30 ppm or less.

5. A device for decontamination by discharging a decontamination solution toward the inside of a room to be decontaminated as a mist for decontamination to decontaminate the inside of the room to be decontaminated, comprising
a decontamination solution supplying device, a mist generator and a mist discharge port, wherein
said decontamination solution supplying device reserves the decontamination solution according to claim 4 and supplies the decontamination solution to said mist generator,
the mist generator converts said decontamination solution into a mist to generate a mist for decontamination, and
the mist discharge port is opened to an internal wall surface of said room to be decontaminated to discharge said mist for decontamination toward the inside of said room to be decontaminated.

6. A method for decontaminating the inside of a room to be decontaminated, comprising a decontamination step and an aeration step, wherein
in said decontamination step, using the device for decontamination according to claim 5, said mist generator is allowed to generate said mist for decontamination from said decontamination solution and the mist for decontamination is discharged from said mist discharge port to decontaminate the inside of said room to be decontaminated, and
in said aeration step after said decontamination step, clean air is introduced into the inside of said room to be decontaminated to discharge a mist and a condensed film of said decontamination solution residual inside said room to be decontaminated toward the outside of said room to be decontaminated.
